(19) Europäisches Patentamt

European Patent Office

Office européen des brevets

(11) EP 1 335 019 A1

(12) EUROPEAN PATENT APPLICATION
published in accordance with Art. 158(3) EPC

(43) Date of publication:
13.08.2003 Bulletin 2003/33

(51) Int Cl.7: C12N 5/10, C12N 15/11,
C12Q 1/02, C12Q 1/70,
A61P 31/18

(21) Application number: 01982814.4

(22) Date of filing: 16.11.2001

(86) International application number:
PCT/JP01/10018

(87) International publication number:
WO 02/040648 (23.05.2002 Gazette 2002/21)

(84) Designated Contracting States:
AT BE CH CY DE DK ES FI FR GB GR IE IT LI LU
MC NL PT SE TR
Designated Extension States:
AL LT LV MK RO SI

(30) Priority: 17.11.2000 JP 2000350685

(71) Applicant: Takeda Chemical Industries, Ltd.
Osaka-shi, Osaka 541-0045 (JP)

(72) Inventors:
• MIYAKE, Hiroshi
  Kagoshima-shi, Kagoshima 891-0175 (JP)
• IIZAWA, Yuji
  Muko-shi, Kyoto 617-0002 (JP)
• BABA, Masanori
  Kagoshima-shi, Kagoshima 891-0103 (JP)

(74) Representative: Rickard, Timothy Mark Adrian
Takeda Euro IP Department,
11-12 Charles II Street
London SW1Y 4QU (GB)

(54) **T CELL LINE AND USE THEREOF**

(57) The present invention provides a T cell line carrying a reporter gene that contains an LTR sequence of HIV and expressing CCR5. This T cell line is suitable for use in an efficient screening method for efficiently finding out a medicine such as an anti-HIV agent.

EP 1 335 019 A1

**Description**

Technical Field

The present invention provides (1) a T cell line (preferably a human T cell line) carrying a reporter gene that contains an LTR sequence of HIV and expressing CCR5, (2) a method of measuring i) the efficiency of infection with HIV or ii) the efficiency of membrane fusion reaction in which an HIV envelope glycoprotein is involved, which comprises using the cell line, (3) a method of screening i) a compound that changes the efficiency of infection with HIV or ii) a compound that changes the efficiency of membrane fusion reaction in which an HIV envelope glycoprotein is involved or iii) a compound that acts on HIV LTR, which comprises using the cell line, and (4) a compound obtained by the screening method and a pharmaceutical composition comprising the compound.

Background Art

The human immunodeficiency virus (HIV) is a retrovirus whose genetic information is encoded by ribonucleic acid (RNA) and known to be a virus causing acquired immunodeficiency syndrome (AIDS). HIV is classified into type 1 and type 2 depending on a difference in their nucleotide sequences. There are a larger number of patients infected with the human immunodeficiency virus type 1 (HIV-1) than patients with the type 2, and HIV-1 also has stronger pathogenicity than the type 2. Thus HIV-1 has been studied selectively, and its life cycle is understood in detail. Entry of HIV-1 into targeted cells is initiated by binding of the viral envelope glycoproteins to CD4 (cluster of differentiation 4) and specific chemokine receptors on the surface of targeted cells, and ultimately established by fusion of the viral envelope membrane with the cell membrane of the targeted cells and the subsequent release of the viral cores into the cells. After the virus entered the targeted cell, the viral own genetic information is converted from RNA into deoxyribonucleic acid (DNA) by using the viral own reverse transcriptase, and the viral DNA is then integrated into the host genomic DNA by the viral own integrase to become a provirus. The provirus transcripts and translates its sequence by using enzymes of the host cell. Thus matured viral particles form and then bud off from the cell finally. In the body of a patient infected with HIV-1, the number of viral particles budded off from one infected cell is estimated at about 1000, and therefore, it is considered that HIV-1 is a virus having a great ability to replicate. Thus HIV-1 replicates by repeating the life cycle consisting of entry, reverse transcription, integration, transcription, translation, particle formation and budding, and it is known that there is considerable genetic diversity of HIV-1 due to poor accuracy of the reverse transcription reaction. This genetic diversity is a major cause for the appearance of a virus (resistant virus) against which an HIV-1 growth inhibitor (anti-HIV-1 agent) is ineffective.

Because a substance that inhibits the replication of HIV, particularly HIV-1 (anti-HIV-1 agent) is expected to prevent the onset of AIDS in patients infected with HIV-1, screening thereof is widely performed. Up to now, reverse transcriptase inhibitors and inhibitors (protease inhibitors) of HIV-1-derived proteolytic enzyme involved in the formation of particles were clinically administered to patients infected with HIV-1, and confirmed to inhibit the replication of HIV-1. As described above, however, there are reported cases where an anti-HIV-1 agent became ineffective due to the appearance of a resistant virus resulting from the genetic diversity of HIV-1. It is also possible that all existing anti-HIV-1 agents become ineffective, and therefore there is a strong demand for development of anti-HIV-1 agents having a novel mode of action.

HIV-1 is divided roughly into two groups depending on the type of a chemokine receptor utilized in infection, that is, CCR5-tropic (R5) HIV-1 that utilizes CD4 and CC chemokine receptor 5 (CCR5) and CXCR4-tropic (X4) HIV-1 that utilizes CD4 and CXC chemokine receptor 4 (CXCR4). After infection with HIV-1, an asymptomatic state continues for a few to ten or more years until the onset of AIDS, and it is known that R5 HIV-1 is mainly isolated in the asymptomatic stage, while X4 HIV-1 is mainly isolated in the AIDS stage. HIV-1 is known to replicate actively in even the asymptomatic stage, and it is expected that the onset of AIDS can be prevented more effectively by inhibiting the replication of HIV-1 in this stage, and thus screening of compounds that can inhibit the replication of R5 HIV-1 is performed very widely.

However, cell lines cultured in laboratories generally do not express a sufficient amount of CCR5 and therefore can not be sufficiently infected with R5 HIV-1 (Lijun Wu et al., Journal of Experimental Medicine, 185, 1681-1691 (1997)). Accordingly, preparation of R5 HIV-1 and various experiments for R5 HIV-1 infection are performed using peripheral blood mononuclear cells prepared from blood-donating volunteers. However, the rate of replication of HIV-1 in the infected cells varies depending on blood donors and thus the reproducibility of infection experiments is not so high, which is preventing efficient screening of anti-R5 HIV-1 agents.

HIV continues to replicate in activated T cells at lymph nodes etc. in an asymptomatic stage for several years to about 10 years after infection. For development of an anti-HIV agent, therefore, it is important to examine the effect of the agent in an HIV multi-round infection system using T cells expressing CCR5. However, conventional established T cell lines are poor in expression of CCR5 and cannot be infected with R5 HIV. Further, though a non-T cell line expressing CCR5, for example MAGI-CCR5 cells, is known and can be infected with HIV, HIV cannot proliferate in these cells. Accordingly, a plurality of groups including our group prepared and reported human T cell lines expressing CCR5 (Baba, M., et al., AIDS Research and Human Retroviruses, 16, 935-941 (2000)). Such cell lines are sensitive to R5 HIV and usable in screening of compounds inhibiting the replication of R5 HIV, but are not suitable for efficient screening because the replication of HIV has to be detected by ELISA. For efficient detection of the replication of HIV, T cell lines carrying a reporter gene activated by HIV Tat (trans-acting transcriptional activator) [Sodroski, J., et al., Science, 229, 74-77 (1985)] are generally used, but such T cell lines do not express CCR5 and are thus not sensitive to R5 HIV (Gervaix, A., et al., Proceedings of the National Academy of Sciences USA, 94, 4653-4658 (1997)).

At present, a T cell line expressing CCR5 and carrying a reporter gene activated by HIV Tat is not known. Accordingly, if a T cell line expressing CCR5 and carrying a reporter gene activated by HIV Tat can be established, efficient screening of a substance that inhibits the replication of R5 HIV-1 can be carried out with high reproducibility, and an evaluation system wherein HIV repeats the life cycle described above and multi-round infection occurs in the T cell line can be constructed, thereby enabling efficient and highly sensitive screening of an anti-HIV agent in an environment similar to in vivo infection of HIV against targeted cells. By this efficient and highly reproducible screening, the probability that an anti-R5 HIV-1 agent will be found is increased, and industrially significant advantages can be brought about.

Summary of Invention

The object of the present invention is to provide a T cell line carrying a reporter gene that contains an LTR sequence of HIV and expressing CCR5, to separate and produce the cell line, and to provide a method of screening with the T cell line.

In light of the problem described above, the present inventors made extensive study and succeeded in establishing a T cell line carrying a reporter gene that contains an LTR (long terminal repeat) sequence of HIV and expressing CCR5, and as a result of further study, the present invention was completed.

That is, the present invention relates to:

(1) a T cell line carrying a reporter gene that contains an LTR sequence of HIV and expressing CCR5;
(2) the T cell line according to the above-mentioned (1), wherein the LTR sequence is a nucleotide sequence identical or substantially identical with the nucleotide sequence set forth in SEQ ID NO:1;
(3) the T cell line according to the above-mentioned (1), wherein the reporter gene is an enzyme gene;
(4) the T cell line according to the above-mentioned (3), wherein the enzyme is alkaline phosphatase;
(5) the T cell line according to the above-mentioned (1), wherein the T cell line is a human-derived cell line;
(6) the T cell line according to the above-mentioned (1), wherein the T cell line is derived from a MOLT-4/CCR5 cell line (FERM BP-7060);
(7) the T cell line according to the above-mentioned (1), which is a MOLT-4/CCR5/LTR-SEAP cell line (FERM BP-7350);
(8) a method of measuring the efficiency of infection with HIV, which comprises using the T cell line according to the above-mentioned (1);
(9) the method according to the above-mentioned (8), wherein the HIV is HIV-1;
(10) the method according to the above-mentioned (8), wherein the HIV is R5 HIV-1;
(11) the method according to the above-mentioned (8), wherein the HIV is HIV present in a clinical sample;
(11a) the method according to the above-mentioned (8), wherein the HIV is HIV-1 present in a clinical sample;
(11b) the method according to the above-mentioned (8), wherein the HIV is R5 HIV-1 present in a clinical sample;
(12) a method of screening a compound that changes the efficiency of infection with HIV, which comprises culturing the T cell line according to the above-mentioned (1) in the presence of a test compound and assaying (preferably, quantitatively determining) a change in the efficiency of infection with HIV;
(13) a method of assaying (preferably, quantitatively determining) the reactivity for cell membrane fusion induced by mixing and culturing (1) the T cell line according to the above-mentioned (1) and (2) a cell expressing (i) an HIV envelope glycoprotein and (ii) a transcription factor for activating HIV LTR;
(14) a method of screening a compound that changes the reactivity for cell membrane fusion induced by mixing and culturing (1) the T cell line according to the above-mentioned (1) and (2) a cell expressing (i) an HIV envelope glycoprotein and (ii) a transcription factor (transcription activator) for activating HIV LTR, which comprises assaying (preferably, quantitatively determining) the reactivity for cell membrane fusion induced by mixing and culturing the cells in the presence of a test compound;
(15) a method of screening a compound that acts on HIV LTR, which comprises using the T cell line according to the above-mentioned (1);
(16) a method of screening a compound that inhibits activated HIV LTR, which comprises using the T cell line according to the above-mentioned (1);

(17) the method according to the above-mentioned (16), wherein the HIV LTR is HIV LTR activated by HIV Tat;

(18) the method according to the above-mentioned (16), wherein the HIV LTR is HIV LTR activated by NFκB;

(19) a compound that changes the efficiency of infection with HIV, which is obtained by the method according to the above-mentioned (12);

(20) the compound according to the above-mentioned (19), which inhibits the efficiency of infection with HIV;

(21) the compound according to the above-mentioned (19), which promotes the efficiency of infection with HIV;

(22) a compound that changes the reactivity for cell membrane fusion, which is obtained by the method according to the above-mentioned (14);

(23) the compound according to the above-mentioned (22), which inhibits cell membrane fusion;

(24) the compound according to the above-mentioned (22), which promotes cell membrane fusion;

(25) a compound that acts on HIV LTR, which is obtained by the method according to the above-mentioned (15);

(26) the compound according to the above-mentioned (25), which activates HIV LTR;

(27) the compound according to the above-mentioned (25), which inhibits HIV LTR;

(28) a compound that inhibits activated HIV LTR, which is obtained by the method according to the above-mentioned (16);

(29) a compound that inhibits activated HIV LTR, which is obtained by the method according to the above-mentioned (17);

(30) a compound that inhibits activated HIV LTR, which is obtained by the method according to the above-mentioned (18);

(31) an agent for promoting the efficiency of infection with a retrovirus vector, which comprises the compound according to the above-mentioned (21), (24) or (26);

(32) the agent according to the above-mentioned (31), wherein the retrovirus vector is derived from HIV;

(33) a pharmaceutical composition comprising the compound according to any one of the above-mentioned (19), (20), (22), (23), (25), (27), (28), (29) and (30);

(34) the composition according to the above-mentioned (33), which is an anti-HIV agent;

(35) the composition according to the above-mentioned (33), which is an agent for preventing or treating AIDS;

(36) use of the compound according to any one of the above-mentioned (19), (20), (22), (23), (25), (27), (28), (29) and (30), or a salt thereof, for the production of an agent for preventing or treating AIDS;

(37) a method of preventing or treating AIDS, which comprises administering a pharmacologically effective amount of the compound according to any one of the above-mentioned (19), (20), (22), (23), (25), (27), (28), (29) and (30) or a salt thereof;

(38) a method of evaluating the effect of combination use of two or more compounds having an anti-HIV activity; and the like.

Brief Description of Drawings

In Fig. 1, an HIV-1 Tat expression vector or a control vector was transiently introduced into a MOLT-4/CCR5/LTR-SEAP cell line, and then the alkaline phosphatase activity in a culture supernatant was measured. The luminescence (relative light unit, RLU) of alkaline phosphatase per second in 1 μl of a culture supernatant is shown on the ordinate.

Fig. 2 shows a flow cytometer chart after an anti-CCR5 antibody was bound to a MOLT-4/CCR5/LTR-SEAP cell line.

Fig. 3 shows a flow cytometer chart after a control antibody was bound to a MOLT-4/CCR5/LTR-SEAP cell line.

Fig. 4 is a graph showing the amount of p24 antigen and the activity of alkaline phosphatase in a culture supernatant of a MOLT-4/CCRS/LTR-SEAP cell line infected with an HIV-1 BaL strain.

Fig. 5 is a graph showing the amount of p24 antigen and the activity of alkaline phosphatase in a culture supernatant of a MOLT-4/CCR5/LTR-SEAP cell line infected with an HIV-1 3B strain.

Mode for Carrying Out the Invention

The T cell line carrying a reporter gene that contains an LTR sequence of HIV and expressing CCR5 according to the present invention can be produced by introducing a reporter gene that contains an LTR sequence of HIV into a T cell line expressing CCR5 (preferably a MOLT-4/CCR5 cell line etc.). The T cell line (preferably a human-derived T cell line etc.) used in the present invention includes, for example, an immortalized T cell line obtained by cloning tumorigenic T cells such as human leukemia T cells. The cloning is carried out according to various known methods. Such methods include, specifically, in view of the permanent proliferation of tumorigenic tissues, a method of cloning immortalized cells from a human leukemia T cell line. The immortalized T cell line thus obtained is transformed by a method known per se so as to express a CCR5 gene in the cells, whereby a T cell line expressing CCR5 can be produced. Specifically, plasmid pcDNA3.1-CCR5 described in Proceedings of the National Academy of Sciences USA), 96: 5698-5703 (1999) is used to transform the immortalized T cell line. This transformation can be carried out in a method known per se, for example, general techniques described in a manual of Sambrook et al. (Molecular Cloning: A Laboratory Manual, Cold Spring Harbor Laboratory Press).

Preferable examples of the T cell line include a MOLT-4 cell line described in Journal of the National Cancer Institute, 49, 891-895 (1972), etc. and preferable examples of the T cell line expressing CCR5 includes "a T cell line having an ability to be infected with CCR5-tropic human immunodeficiency virus" obtained by transforming an immortalized T cell strain such as a MOLT-4 clone 8 cell strain (Journal of Virology, March, 1986, pp. 1159-1162) in a method known per se, and more specifically a MOLT-4/CCR5 (FERM BP-7060) strain obtained in Reference Example 1 described later.

The T cell line expressing CCR5 thus obtained is transformed by a method known per se to be introduced a reporter gene that contains an LTR sequence of HIV [wherein, the LTR sequence may be either a complete or partial nucleotide sequence, preferably a partial nucleotide sequence [e.g., a sequence described in Kimpton, J. et al. in Journal of Virology, 66, 2232-2239 (1992)], more preferably a nucleotide sequence set forth in SEQ ID NO:1 described later (i.e. 5'-cttcaagaactgctgacatcgagcttgctacaagggactttccgctggggactttccag ggaggcgtggcctgggcgggactggggagt-ggcgagccctcagatgctgcatataagca gctgcttttgcctgtactgggtctctctggttagaccagatctgagcctgggagctct ctggctaactagggaac-ccactgcttaagcctcaataaagctt-3')], whereby the T cell line carrying a reporter gene and expressing CCR5 of the present invention can be produced. A preferable partial nucleotide sequence of the LTR sequence may be identical with the nucleotide sequence of SEQ ID NO:1, but any nucleotide sequences achieving the same effect as that of the nucleotide sequence of SEQ ID NO:1 (i.e., nucleotide sequences substantially identical with the nucleotide sequence of SEQ ID NO:1) may be used, and examples of such nucleotide sequences include a nucleotide sequence wherein one or more bases are substituted or deleted in the nucleotide sequence of SEQ ID NO:1 or a nucleotide sequence wherein one or more bases are added to the nucleotide sequence of SEQ ID NO:1.

Examples of the reporter gene that contains an LTR sequence of HIV include LTR-SEAP (secreted form of human placental alkaline phosphatase), LTR-β galactosidase etc. As the reporter gene, genes for β-galactosidase, chloramphenicol acetyl transferase, luciferase, alkaline phosphatase, human growth hormone, green fluorescence protein, etc., among which preferably genes for enzymes such as β-galactosidase, chloramphenicol acetyl transferase, luciferase and alkaline phosphatase are used, and the enzyme is preferably alkaline phosphatase or the like. In particular, LTR-SEAP or the like is used preferably as the reporter gene that contains an LTR sequence of HIV. Preferable examples of the T cell line carrying the reporter gene that contains an LTR sequence of HIV and expressing CCR5 include the MOLT-4/CCR5/LTR-SEAP (FERM BP-7350) cell line obtained in Example 1 described later.

The T cell line thus obtained can be subjected to a wide variety of screening by culturing it in a medium used in culture of T cell lines (for example, RPMI1640 medium etc.), and usually the culture temperature is about 15°C to about 55°C, preferably about 20°C to about 45°C, more preferably about 25°C to about 40°C. The culture time is about 1 hour to about 6 months, preferably about 1.5 hours to about 60 days. The culture is carried out preferably under an atmosphere such as $CO_2$ gas. The concentration of $CO_2$ gas is about 3 to 10 (V/V) %.

The T cell line carrying a reporter gene that contains an LTR sequence of HIV and expressing CCR5 according to the present invention can be used together with a test compound and/or HIV as necessary to perform (I) measurement and screening of the efficiency of infection with HIV, (II) assay (preferably quantitative determination) of the reactivity and screening of the efficiency of reaction for membrane fusion in which an HIV envelope glycoprotein is involved, (III) screening of a compound that changes (e.g. promotes or inhibits) the efficiency of infection with HIV, (IV) screening of a compound that changes (e.g. promotes or inhibits) the efficiency of reaction of membrane fusion in which an HIV envelope glycoprotein is involved, and (V) screening of a compound that acts on HIV LTR (e.g. activates HIV LTR, inhibits HIV LTR or inhibits activated HIV LTR).

The test compound includes, for example, peptides, proteins, non-peptide compounds, synthetic compounds, fermentation products, cell extracts, plant extracts, animal tissue extracts, serums etc., and these test compounds may be known or novel compounds, or may be produced by deriving from known compounds by a known method.

Hereinafter, the respective methods are described.

(I) Measurement and screening of the efficiency of infection with HIV

The T cell line carrying a reporter gene that contains an LTR sequence of HIV and expressing CCR5 is infected with HIV by a method known per se. That is, the T cell line carrying a reporter gene that contains an HIV LTR sequence and expressing CCR5 is cultured with HIV in a medium used for culturing T cell lines (e.g., a medium such as RPMI1640). HIV refers to an experimental strain used generally in laboratories, a clinically isolated strain obtained from patients infected with HIV, and HIV contained in a clinical sample (e.g., blood etc.) derived from patients infected with HIV (preferably HIV-1, more preferably R5 HIV-1, still more preferably HIV present in a clinical sample; the HIV present in a clinical sample is preferably HIV-1 present in a clinical sample, more preferably R5 HIV-1 present in a clinical sample). The culture temperature is about 15°C to about 55°C, preferably about 20°C to about 45°C, more preferably about 25°C to about 40°C. The culture time is about 1 hour to about 2 months, preferably about 1.5 hours to about 30 days, more preferably about 2 hours to about 20 days. The culture is carried out under a $CO_2$ atmosphere. The concentration of $CO_2$ is about 3 to 10%(V/V).

Establishment of infection with human immunodeficiency virus can be detected by measuring an increase in the amount of HIV in a culture supernatant of the T cell line. The measurement of the amount of HIV is carried out by a method known per se, that is, 1) a method of measuring the amount of HIV RNA by the RT-PCR method, 2) a method of measuring the amount of p24 antigen by enzyme immunoassay (e.g., a method of using a Retro-Tek HIV-1 p24 Antigen ELISA kit, manufactured by Zepto Metrix, USA), 3) a method of measuring the enzyme activity of HIV reverse transcriptase, or 4) a method of measuring the amount or activity of a reporter protein, preferably by measuring the activity of a reporter enzyme, more preferably by measuring the activity of a reporter enzyme in a culture supernatant, still more preferably by measuring the activity of alkaline phosphatase in a culture supernatant by a method known per se (e.g., coloration method, luminescence method or fluorescence method).

Here, HIV present in a clinical sample can be used to judge, for example, whether HIV having an ability to infect is present in body fluid (e.g., blood, seminal fluid etc.) of an antibody-positive patient.

(II) Assay of the reactivity and screening of the efficiency of reaction for membrane fusion in which an HIV envelope glycoprotein is involved

The reactivity for cell membrane fusion induced by mixing and culturing (1) the T cell line carrying a reporter gene that contains an LTR sequence of HIV and expressing CCR5 with or in the presence of a test compound with (2) a cell expressing (i) an HIV envelope glycoprotein and (ii) a transcription factor for activating HIV LTR can be assayed by detecting (preferably determining, more preferably quantitatively determining) the reporter activity, and the reporter activity also can be applied to screening of the efficiency of reaction. Preferably, the reporter activity is detected (preferably determined, more preferably quantitatively determined) without preparing an extract of the fused cells (for example, without isolating an extract of the fused cells), and more preferably the alkaline phosphatase activity in the culture supernatant is detected by a method known per se (e.g., a coloration method, luminescence method and fluorescence method).

(II-a) Preparation of the cell expressing (i) an HIV envelope glycoprotein and (ii) a transcription factor for activating HIV LTR (e.g., Tat etc.)

The HIV envelope glycoprotein may be an HIV envelope glycoprotein derived from either HIV-1 or HIV-2 and also derived from R5, X4 or R5X4 HIV as chemokine receptor-tropic HIV. Preferably an HIV envelope glycoprotein derived from HIV-1, more preferably an HIV envelope glycoprotein derived from a HIV-1 strain (R5 HIV-1) utilizing CCR5, and still more preferably an HIV envelope glycoprotein derived from a R5 HIV-1 JR-FL strain [Koyanagi, Y., et al., Science, 236, 819-822 (1987)] are used. Either a plasmid vector or a virus vector can be used for transient expression of an HIV envelope glycoprotein and a transcription factor for activating HIV LTR (e.g., Tat etc.). Introduction of the gene can be carried out in a method known per se, and for example, a calcium phosphate method, a lipofection method, an electroporation method or a DEAE dextran method, etc. can be used.

The cell line expressing an HIV envelope glycoprotein and a transcription activation factor (e.g., Tat etc.) is a cell line derived from mammals (e.g., guinea pigs, mice, cats, dogs, pigs, sheep, cows, monkeys, humans etc., preferably humans). A cell line to which genes can be introduced efficiently and wherein the genes can be expressed efficiently is preferably used, and preferable examples of such a cell line include a cell line having SV40 large T antigen. Such a particularly preferable example is 293T, a cell line derived from human fetal kidney [DuBridge, R.B., et al., Molecular and Cellular Biology, 7, 379-387 (1987)].

Preparation of 293T cells expressing an HIV envelope glycoprotein and a transcription activation factor (e.g., Tat etc.) can be carried out by a method known per se. Specifically, the 293T cells can be prepared by the following method or its analogous method.

First, about $0.1 \times 10^6$ cells to about $2 \times 10^6$ cells, preferably about $0.5 \times 10^6$ cells to about $1.5 \times 10^6$ cells, more preferably about $1 \times 10^6$ cells per 10 cm$^2$ of a culture dish are plated and cultured overnight (37°C, 5%$CO_2$) in a medium (e.g., Dulbecco's modified Eagle medium (DMEM) supplemented with 10%(v/v) inactivated fetal bovine serum (ICN) and penicillin-streptomycin at such concentrations as to be 100 U/ml and 100 μg/ml as penicillin G sodium and streptomycin sulfate, respectively). On the next day, the culture solution is removed, and a previously prepared DNA-lipid solution is added thereto, and the cells are cultured at 37°C in 5% $CO_2$ for about 2 hours to about 10 hours, preferably for about 4 hours to about 8 hours, more preferably for about 6 hours. After culture, the DNA-lipid solution is removed, and after the medium is added, the cells are further cultured at 37°C in 5% $CO_2$ for about 12 hours to about 4 days, preferably for about 1 day to about 3 days, more preferably for about 2 days.

The DNA-lipid solution can be prepared by mixing a DNA solution with a lipid solution and incubating the mixture at room temperature (about 15 to 35°C) for about 5 minutes to about 2 hours, preferably about 15 minutes to about 1 hour, more preferably for about 30 minutes. The DNA solution can be prepared by adding a DNA to a DMEM medium or OPTI-MEM medium (Gibco) not containing additives such as inactivated fetal bovine serum. The medium is used in an amount of about 10 μl to about 1000 μl, preferably about 50 μl to about 500 μl, more preferably about 100 μl per 10 cm$^2$ of a culture dish. The DNA is a mixture of an Env [envelope glycoprotein] expression vector, a Rev [regulation of virion-protein expression] expression vector and a transcription activation factor expression vector (e.g., a Tat expression vector etc.), and per 10 cm$^2$ of a culture dish, the Env expression vector is used in an amount of about 0.1 μg to about 10 μg, preferably about 0.2 μg to about 2 μg, more preferably about 0.6 μg; the Rev expression vector is used in an amount of about 0.01 μg to about 1 μg, preferably about 0.05 μg to about 0.5 μg, more preferably about 0.2 μg; and the transcription activation factor expression vector (e.g., a Tat expression vector etc.) is used in an amount of about 0.1 μg to about 10 μg, preferably about 0.2 μg to about 2 μg, more preferably about 1.0 μg. The proportion of their amounts is preferably about 0.6 : 0.2 : 1.0.

The lipid solution can be prepared by adding a lipid reagent (e.g., lipofectamine (Gibco) etc.) to a DMEM medium or OPTI-MEM medium (Gibco) not containing additives such as inactivated fetal bovine serum. The medium is used in an amount of about 10 μl to about 1000 μl, preferably about 50 μl to about 500 μl, more preferably about 100 μl per 10 cm$^2$ of a culture dish. The lipid reagent is used in an amount of about 0.1 μl to about 100 μl, preferably about 1 μl to about 10 μl, more preferably about 6 μl, per 10 cm$^2$ of a culture dish.

(III) Screening of a compound that changes the efficiency of infection with HIV

The T cell line carrying a reporter gene that contains an LTR sequence of HIV and expressing CCR5 is infected with HIV in the presence of a test compound. The amount of HIV in the culture supernatant is measured by the method described above, and a compound that changes the increases in the amount of HIV is selected, whereby a compound that changes the efficiency of infection with HIV can be screened.

(IV) Screening of a compound that changes the efficiency of reaction of membrane fusion in which an HIV envelope glycoprotein is involved

A compound that changes the reactivity for cell membrane fusion in which an HIV envelope glycoprotein is involved can be screened by quantitatively determining each reporter activity in the case (i) wherein (1) the T cell line carrying a reporter gene that contains an LTR sequence of HIV and expressing CCR5 and (2) the cell expressing (i) an HIV envelope glycoprotein and (ii) a transcription factor for activating HIV LTR are mixed and cultured in the absence of a test compound and in the case (ii) wherein (1) the T cell line carrying a reporter gene that contains an LTR sequence of HIV and expressing CCR5 and (2) the cell expressing (i) an HIV envelope glycoprotein and (ii) a transcription factor for activating HIV LTR are mixed and cultured in the presence of a test compound, and thereby comparing a change in the reactivity or the efficiency of reaction for cell membrane fusion induced in the two cases; in each case, a change in the reactivity for cell membrane fusion is assayed preferably by quantitatively determining the reporter activity without preparing an extract of the fused cells (for example, without isolating an extract of the fused cells), more preferably by detecting the alkaline phosphatase activity in the culture supernatant by a method known per se (e.g., a coloration method, luminescence method and fluorescence method).

(V) Screening of a compound that acts on HIV LTR

The compound that acts on HIV LTR refers to i) a compound that activates HIV LTR, ii) a compound that inhibits HIV LTR, and iii) a compound inhibiting activated HIV LTR. The activated HIV LTR refers to HIV LTR activated by HIV Tat, NFκB or the like.

The T cell line carrying a reporter gene that contains an LTR sequence of HIV and expressing CCR5, or said cell line wherein the carried LTR is activated, is cultured under the same conditions as described above in the presence of a test compound, and then its reporter activity (preferably the reporter activity in the culture supernatant) is measured, whereby a compound that acts on HIV LTR can be screened.

The test compound that may be obtained by using the screening method of the present invention is a compound selected from the above-mentioned test compounds, for example, peptides, proteins, non-peptide compounds, synthetic compounds, fermentation products, cell extracts, plant extracts, animal tissue extracts, plasmas etc., and includes a compound that changes the efficiency of infection with HIV; a compound that changes the efficiency of reaction for membrane fusion in which an HIV envelope glycoprotein is involved; a compound that changes the reactivity for cell membrane fusion induced by mixing (1) the T cell line carrying a reporter gene that contains an LTR sequence of HIV and expressing CCR5 with (2) the cell expressing (i) an HIV envelope glycoprotein and (ii) a transcription factor for activating HIV LTR, and then culturing the mixture; a compound that acts on HIV LTR and the like.

Examples thereof include, for example:

a compound that inhibits the efficiency of infection with HIV;
a compound that promotes the efficiency of infection with HIV;
a compound that inhibits cell membrane fusion;
a compound that promotes cell membrane fusion;
a compound that activates HIV LTR;
a compound that inhibits HIV LTR; and
a compound that inhibits activated HIV LTR.

The compound that promotes the efficiency of infection with HIV or cell membrane fusion is also useful as a reagent for promoting the efficiency of infection with a retrovirus vector (preferably, an HIV-derived retrovirus vector).

The compound that may be obtained by the screening method of the present invention (also referred to hereinafter as the compound of the present invention) may be a prodrug, or may have formed a salt or a hydrate. The prodrug is not particularly limited insofar as it is a compound converted into the compound of the present invention by reaction with an enzyme or gastric acid etc. under physiological conditions in a living body, that is, a compound converted into the compound of the present invention by enzymatic oxidation, reduction, hydrolysis etc. or a compound converted into the compound of the present invention by hydrolysis with gastric acid etc.

The prodrug of the compound of the present invention includes those compounds wherein an amino group of the compound of the present invention is acylated, alkylated or phosphorylated (for example, those compounds wherein an amino group of the compound of the present invention is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, or tert-butylated); those compounds wherein a hydroxyl group of the compound of the present invention is acylated, alkylated, phosphorylated or borated (for example, those compounds wherein a hydroxyl group of the compound of the present invention is acetylated, palmitoylated, propanoylated, pivaloylated, succinylaed, fumarylated, alanylated or dimethylaminomethylcarbonylated); and those compounds wherein a carboxyl group of the compound of the present invention is esterified or amidated (for example, those compounds wherein a carboxyl group of the compound of the present invention is ethylesterified, phenylesterified, carboxyoxymethylesterified, dimethylaminomethylesterified, pivaloyloxymethylesterified, ethoxycarbonyloxyethylesterified, phthalidylesterified, (5-methyl-2-oxo-1,3-dioxolen-4-yl)methylesterified, cyclohexyloxycarbonylethylesterified or methylamidated). These compounds can be produced from the compound of this invention in a method known per se.

Further, the prodrugs of the compound of the present invention may be a compound converted into the compound of the present invention under those physiological conditions described in "Iyakuhin No Kaihatsu" (Development of Pharmaceutical Preparations), Vol. 7, Molecular Design, pp. 163-198, published in 1990 by Hirokawa Shoten.

The compound which may be obtained by the screening method of the present invention may have formed salts, and such salts include pharmacologically and physiologically acceptable salts such as a salt with an inorganic base, a salt with an organic base, a salt with an inorganic acid, a salt with an organic acid, and a salt with a basic or acidic amino acid. Preferable examples of the salt with an inorganic base include alkali metal salts such as sodium salt, potassium salt etc.; alkaline earth metal salts such as calcium salt, magnesium salt etc.; and aluminum salts, ammonium salts etc. Preferable examples of the salt with an organic base include salts with trimethylamine, triethylamine, pyridine, picoline, ethanolamine, diethanolamine, triethanolamine, dicyclohexylamine, N,N'-dibenzylethylenediamine etc. Preferable examples of the salt with an inorganic acid include salts with hydrochloric acid, hydrobromic acid, nitric acid, sulfuric acid, phosphoric acid, etc. Preferable examples of the salt with an organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid etc. Preferable examples of the salt with a basic amino acid include salts with arginine, lysine, ornithine etc., and preferable examples of the salt with an acidic amino acid include salts with aspartic acid, glutamic acid etc.

The compound that may be obtained by the screening method of the present invention (preferably a compound inhibiting the efficiency of infection or cell membrane fusion) is useful as an anti-HIV agent (therapeutic or prophylactic agent for HIV infections) and used particularly preferably as a therapeutic or prophylactic agent for AIDS.

When the compound that may be obtained by the screening method of the present invention is used as the above described therapeutic or prophylactic agent, it can be used in a usual manner. The compound can be administered as it is or as a suitable pharmaceutical composition. The pharmaceutical composition used for the above described administration may contain the above described compound together with pharmaceutically acceptable carriers, diluents or excipients. The composition is provided as a dosage form suitable for oral or parenteral administration.

That is, for example, the composition for oral administration may be in a solid or liquid form, specifically in the form of tablets (including sugar-coated tablets and film-coated tablets), pills, granules, powder, capsules (including soft capsules), syrups, emulsions, suspensions etc. Such composition is produced by a method known per se and contains carriers, diluents or excipients generally used in the field of pharmaceutical manufacturing. For example, the carriers and excipients used in tablets include lactose, starch, sucrose, magnesium stearate etc.

The composition for parenteral administration includes e.g. injections, suppositories etc., and the injections include intravenous injection, subcutaneous injection, intracutaneous injection, intramuscular injection and intravenous infusion. These injections are prepared by a method know per se, for example, by dissolving, suspending or emulsifying the compound in a germ-free, aqueous or oily solution used conventionally for injection. The aqueous solution for injection includes e.g. physiological saline ,an isotonic solution containing glucose and other auxiliary, etc. and may be used in combination with suitable solubilizers such as alcohols (e.g., ethanol etc.), polyalcohols (e.g., propylene glycol, polyethylene glycol) and nonionic surfactants (e.g., Polysorbate 80 and HCO-50 (polyoxyethylene (50 mol) adduct of hydrogenated castor oil) etc. The oily solution includes e.g. sesame oil, soybean oil etc., and may be used in combination with a solubilizer such as benzyl benzoate, benzyl alcohol etc. The prepared injection solution is usually filled in suitable ampoules. The suppositories for administration to rectum are prepared by mixing the above compound with a conventional base for suppositories.

The oral or parenteral pharmaceutical composition described above is prepared conveniently in a unit dosage form adaptable to the amount of an active ingredient administered. Such a unit dosage form includes e.g. tablets, pills, capsules, injections (ampoules), suppositories, etc., where the above compound is contained in an amount of usually 5 to 500 mg per each unit dosage form and preferably 5 to 100 mg/unit for the injections or 10 to 250 mg/unit for the other pharmaceutical forms.

The respective compositions described above may contain any other active ingredients (for example, a reverse transcriptase inhibitor, a protease inhibitor etc.) that do not undesirably interact with the above compound.

The pharmaceutical preparation thus obtained is safe and low toxic so that, for example, it can be administered to mammals (e.g., mouse, rat, rabbit, sheep, pig, cow, horse, bird, cat, dog, monkey, chimpanzee, human etc.).

The dosage of the compound may vary depending on the action of the compound, disease to be treated, subject of administration, administration route etc., and for example, when such compound as a CCR5 antagonist is orally administered for the purpose of treatment of AIDS, its dosage is usually about 0.1 mg to 5 g, preferably about 10 mg to 4 g and more preferably about 100 mg to 3 g per day for an adult (body weight 60 kg).

For parenteral administration, the dosage of the compound may vary depending on the subject of administration, disease to be treated, etc., and for example, when such compound as a CCR5 antagonist is administered by injection for the purpose of treatment of AIDS, it is conveniently about 0.01 mg to 2 g, preferably about 0.1 mg to 1 g and more preferably about 0.1 mg to 500 mg per day of intravenous injection for an adult (body weight 60 kg). The compound of the present invention can also be administered into other animals in an equivalent dosage to the above described dosage for 60 kg body weight.

In the method of evaluating the effect of combination use of two or more compounds having an anti-HIV activity, the "anti-HIV activity" refers to reverse transcriptase inhibition, protease inhibition, integrase inhibition, Tat inhibition, envelope inhibition, receptor inhibition and the like. In this case, "two or more compounds" may be identical or different in respect of their anti-HIV activity. The HIV used in evaluation can be either R5, X4 or R5X4 viruses.

The "method of evaluating the effect of combination use" can be carried out specifically in the same manner as in "(III) Screening of a compound that changes the efficiency of infection with HIV", "(IV) Screening of a compound that changes the efficiency of reaction for membrane fusion in which an HIV envelope glycoprotein is involved", "(V) Screening of a compound that acts on HIV LTR", and the method in Example 3 described later. That is, the effect of combination use can be evaluated by comparing the evaluation result of a single drug with that of combination use of two or more drugs. This method can facilitate evaluation of the effect of combination use of anti-HIV drugs and thus it is clinically significantly useful.

The MOLT-4/CCR5/LTR-SEAP cell line obtained in Example 1 described later has been deposited under the accession No. FERM BP-7350 with the National Institute of Bioscience and Human-Technology (NIBH), the Agency of Industrial Science of Technology, the Ministry of International Trade and Industry, Japan since November 8, 2000 and under the accession No. IFO 50526 with Institute for Fermentation, Osaka (IFO), Japan since October 13, 2000.

The MOLT-4/CCR5 cell line obtained in Reference Example 1 described later has been deposited under the accession No. FERM BP-7060 with the National Institute of Bioscience and Human-Technology (NIBH), the Agency of Industrial Science of Technology, the Ministry of International Trade and Industry since February 29, 2000 and under the accession No. IFO 50521 with Institute for Fermentation, Osaka (IFO) since January 27, 2000.

The following examples are intended to illustrate in detail but not limit the present invention. Unless otherwise specified, techniques of genetic manipulation followed the general techniques described in Sambrook et al., Molecular Cloning: A Laboratory Manual, Cold Spring Harbor Laboratory Press.

Reference Example 1. Establishment of MOLT-4/CCR5 cell line

(1) Determination of the concentration of selective agent (geneticin)

MOLT-4 cells were cultured at 37°C in a 5% $CO_2$ gas in RPMI1640 medium (Life Technology, US) containing 10% fetal bovine serum (FBS, manufactured by ICN, US), 100 U/ml penicillin G (Life Technology, US) and 100 µg/ml streptomycin (Life Technology, US) (referred to hereinafter as MOLT-4 basic medium).

To the MOLT-4 basic medium was added geneticin (Life Technology, US) at a concentration of 100, 200, 300, 400, 500, 600, 700, 800, 900 or 1000 µg/ml, and the cells were cultured at a density of $2\times10^3$ cells/ml for 2 weeks, and the growth of the cells was observed under a microscope to determine the minimum concentration of the agent at which the growth was completely inhibited.

(2) Transfection

To 250 µl of RPMI1640 medium free of FBS, penicillin G and streptomycin was added 15 µl of cellfectin (Life Technology, US) to prepare a lipid solution which then added to each well of a 24-well culture plate.

To 250 µl of additive-free RPMI1640 medium was added 3.2 µg of pcDNA3.1-CCR5 (Baba et al., Proceedings of the National Academy of Sciences, USA, 96:5698-5703 (1999)) to prepare a DNA solution. This DNA solution was mixed with the lipid solution and left at room temperature for 30 minutes, and then 250 µl of DNA-lipid mixed solution was removed from the A1 well.

Fifty microliters of MOLT-4 cells suspended at a density of $8\times10^6$ cells/ml in additive-free RPMI1640 medium were added to each well and cultured at 37°C in a 5% $CO_2$ gas for 4 hours. Further, 600 µl of RPMI1640 medium containing 15% FBS, 100 U/ml penicillin G and 100 µg/ml streptomycin was added thereto, and the cells were cultured for 2 days. The whole volume of the culture solution was transferred to a 100 mm culture dish containing 10 ml of MOLT-4 basic medium with 1 mg/ml geneticin, and continued to culture at 37°C in a 5% $CO_2$ gas with exchanging the medium solution as necessary, to grow geneticin-resistant cells.

(3) Preparation of feeder cells

MOLT-4 cells were suspended at a density of $1\times10^7$ cells/ml in a phosphate buffer (PBS, Life Technology, US), and 200 µl of the cell suspension was pipetted into a sample tube. Twenty microliters of 0.5 mg/ml mitomycin C (Wako Pure Chemical Industries, Ltd., JP) was added thereto and incubated at 37°C for 30 minutes. The cells were washed 5 times with MOLT-4 basic medium and then suspended at a density of $1\times10^5$ cells/ml in MOLT-4 basic medium containing 1 mg/ml geneticin.

(4) Single-cell cloning

The above feeder cells were pipetted into each well of a 96-well cell culture plate at an amount of 100 µl per well. Growing geneticin-resistant cells were suspended at a density of 3 cells/ml in MOLT-4 basic medium containing 1 mg/ml geneticin, and then pipetted into each well of the 96-well cell culture plate to which the feeder cells had been added. The growth of the cells was observed under a microscope, to separate the grown cells, which were then cultured in a larger scale as necessary.

(5) Examination of CCR5 expression

The geneticin-resistant clones obtained in the above (4) were washed twice with a reaction solution [2% (V/V) FBS, 0.1% (W/V) sodium azide (Wako Pure Chemical Industries, Ltd., JP)/phosphate buffer] and then suspended in 40 µl of the same reaction solution. To this suspension was added 10 µl of fluorescein isothiocyanate (FITC)-labeled anti-CCR5 antibody clone 2D7 (Farmingen, US), and the cells were incubated at 4°C for 45 minutes.

The cells were separated and washed 3 times with each 1 ml of the above reaction solution, then suspended in 300 µl of the reaction solution and analyzed for antibody binding by a flow cytometer (JASCO CytoACE-300, JASCO Corporation, JP) to give a MOLT-4/CCR5 cell strain [FERM BP-7060; IFO 50521], which was confirmed to express CCR5 since the peak showing the binding of anti-CCR5 antibody to cells was evidently shifted toward right (toward higher fluorescence intensity) as compared with that of the staring cells, MOLT4 cell strain.

Example 1. Establishment of MOLT-4/CCR5/LTR-SEAP cell line

### (1) Determination of the concentration of selective agent (puromycin)

MOLT-4/CCR5 cells were cultured at 37°C in 5% $CO_2$ gas in RPMI1640 medium (Life Technology, US) containing 10% fetal bovine serum (FBS, manufactured by ICN, US), 100 U/ml penicillin G (Life Technology, US), 100 μg/ml streptomycin (Life Technology, US) and 500 μg/ml G418 sulfate (Life Technology, US) (referred to hereinafter as MOLT-4/CCR5 basic medium).

Puromycin (Sigma, US) was added at a concentration of 0.1, 0.2, 0.5 or 1.0 μg/ml to the MOLT-4/CCR5 basic medium, and the cells were cultured at a density of $2 \times 10^3$ cells/ml for 1 week, and the growth of the cells was observed under a microscope to determine the minimum concentration of the agent at which the growth was completely inhibited.

### (2) Construction of LTR-SEAP

A part of a LTR sequence was amplified by PCR using HIV-1-infected cell-derived genomic DNA as the template, together with primer A: ggagatcttcaagaactgctgacatcgagc (SEQ ID NO:2) and primer B: aggcaagctttattgaggcttaagcagtgg (SEQ ID NO:3). The amplified sequence was digested with restriction enzymes BglII and HindIII and then integrated into pSEAP2-Basic (Clontech) at a BglIII/HindIII site to give LTR-SEAP.

### (3) Transfection

To each well of a 6-well culture dish were added $1 \times 10^6$ cells suspended in 800 μl of OPTI-MEM (Gibco) medium. To the cells was added 200 μl of a DNA-lipid solution, and the cells were cultured at 37°C in 5% $CO_2$ for 4 hours. After the culture, 1 ml of RPMI1640 medium containing 20% FBS was added thereto, and the cells were cultured at 37°C in 5% $CO_2$ for 3 days. The DNA-lipid solution was prepared by mixing a DNA solution with a lipid solution. The DNA solution was prepared by adding 1 μg of LTR-SEAP and 0.1 μg of pPURO (Clontech) to 100 μl of OPTI-MEM medium. The lipid solution was prepared by adding 6 μl of Lipofectamine 2000 (Life Technology, US) to 100 μl of OPTI-MEM medium.

After 3 days, a quarter of the cells was transferred to a 100 mm culture dish containing 10 ml of MOLT-4/CCR5 basic medium with 0.1 μg/ml puromycin and cultured at 37°C in 5% $CO_2$ gas with exchanging the medium as necessary, to grow puromycin-resistant cells.

### (4) Preparation of feeder cells

MOLT-4 cells were suspended at a density of $1 \times 10^7$ cells/ml in a phosphate buffer (PBS, Life Technology, US), and 200 μl of the cell suspension was pipetted into a sample tube. Twenty microliters of 0.5 mg/ml mitomycin C (Wako Pure Chemical Industries, Ltd., JP) was added thereto and incubated at 37°C for 30 minutes. The cells were washed 5 times with MOLT-4/CCR5 basic medium and suspended at a density of $1 \times 10^5$ cells/ml in MOLT-4/CCR5 basic medium containing 0.1 μg/ml puromycin.

### (5) Single-cell cloning

The above feeder cells were pipetted into each well of a 96-well cell culture plate at an amount of 100 μl per well. Growing puromycin-resistant cells were suspended at a density of 3 cells/ml in MOLT-4/CCR5 basic medium containing 0.1 μg/ml puromycin, and then 100 μl of the cell suspension was pipetted (0.3 cell per well) into each well of the 96-well cell culture plate to which the feeder cells had been added. The growth of the cells was observed under a microscope, to separate the grown cells, which were then. cultured in a larger scale as necessary.

### (6) HIV Tat-dependent activation of a reporter

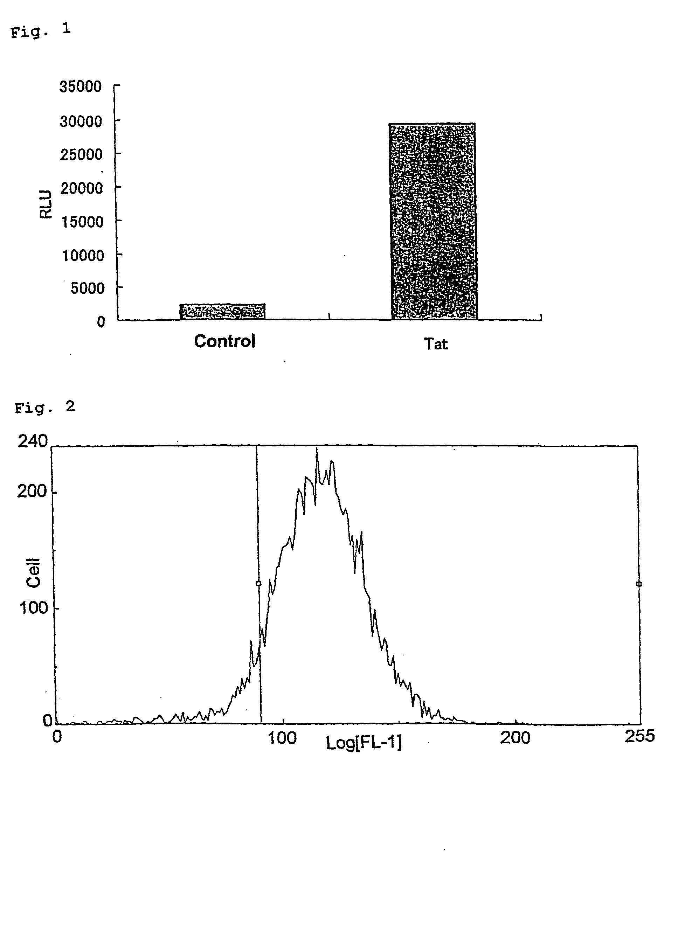

The puromycin-resistant clones obtained in the above (5) were transfected by a known method per se with an HIV Tat expression vector. After culture for 2 days, the alkaline phosphatase activity in the supernatant was measured by Great EscAPe SEAP Chemiluminescence Detection Kits (Clontech). Puromycin-resistant clones whose alkaline phosphatase activity was recognized to be increased by Tat were selected (Fig. 1).

### (7) Confirmation of expression of CCR5 and CD4 on cell surfaces

The puromycin-resistant clones obtained in the above (5) were washed twice with a reaction solution [2% (V/V) FBS, 0.1% (W/V) sodium azide (Wako Pure Chemical Industries, Ltd., JP)/phosphate buffer] and then suspended in 40 µl of the same reaction solution. To this suspension was added 10 µl of fluorescein isothiocyanate (FITC)-labeled anti-CCR5 antibody clone 2D7 (Farmingen, US) or a FITC-labeled control antibody (Pharmingen), and the cells were incubated at 4°C for 45 minutes.

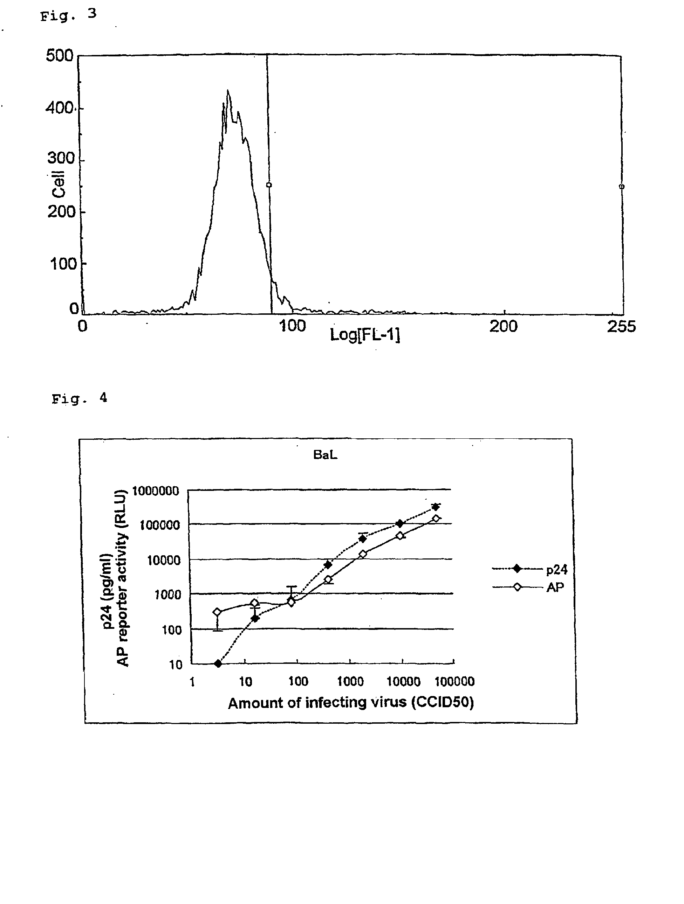

After the reaction, the cells were washed 3 times with 1 ml of the above reaction solution, then suspended in 600 µl of the reaction solution and analyzed for antibody binding by a flow cytometer (JASCO CytoACE-300, JASCO Corporation, JP). The results are shown in Figs. 2 and 3.

The peak showing the binding of the anti-CCR5 antibody to the MOLT-4/CCR5/LTR-SEAP cell line [FERM BP-7350; IFO 50526] (Fig. 2) was evidently shifted toward right (toward higher fluorescence intensity) as compared with the binding of the control antibody (Fig. 3), thus confirming expression of CCR5 on the cell surface of the MOLT-4/CCR5/LTR-SEAP cell line.

Example 2. HIV-1 infection and measurement of the amount of virus antigen and alkaline phosphatase activity

A test was performed to confirm that the alkaline phosphatase activity can be an index of viral growth similarly to the amount of p24 antigen, as described below.

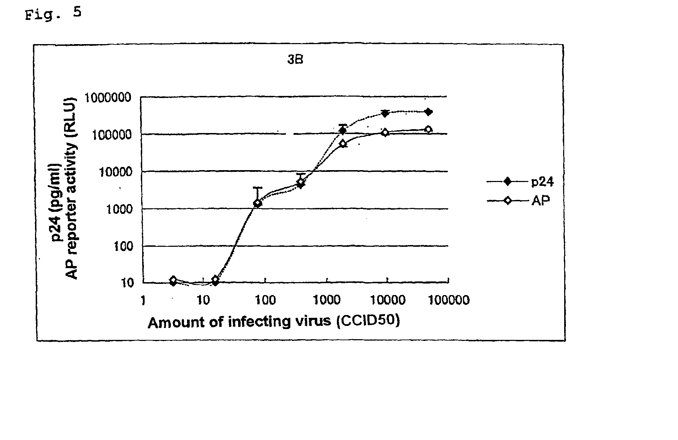

A mixture of $2 \times 10^4$ of MOLT-4/CCR5/LTR-SEAP cells and various $CCID_{50}$ (cell culture infective dose 50%) of viruses was cultured at 37°C in a 5% $CO_2$ gas. On fifth day after the infection, a quarter of the cells was subcultured for 5 days. On the tenth day after the infection, the culture supernatant was recovered, and the amount of p24 antigen and the alkaline phosphatase activity were measured by the following measurement methods. The viruses used were CCR5-tropic HIV-1 BaL strain and CXCR4-tropic HIV-1 3B strain. The experimental results are shown in Fig. 4 (HIV-1 BaL strain) and Fig. 5 (HIV-1 3B strain). In the figures, the amount of p24 antigen (pg/ml) and chemiluminescence (RLU) are shown on the ordinate, and the amount of the virus ($CCID_{50}$/well) is shown on the abscissa. The experiment was carried out using 3 wells, and the average and standard deviation are shown. An increase in the amount of p24 antigen and in the alkaline phosphatase activity depending on the amount of the virus used was recognized, and it was shown that similarly to the amount of p24 antigen, the alkaline phosphatase activity can be an index of viral growth.

1) Measurement of the amount of p24 antigen

The amount of p24 antigen was measured by using a Retro-TEK p24 antigen ELISA kit manufactured by ZeptoMetrix. The culture supernatant diluted suitably with a diluent was added to an ELISA strip previously washed with a washing solution, and allowed to react at 37°C for 2 hours. After washing, a detecting antibody was added thereto and incubated at 37°C for 1 hour. After washing, a secondary antibody was added thereto and incubated at 37°C for 30 minutes. After washing, a coloring substrate was added thereto, incubated at room temperature for 30 minutes, and then the coloration was terminated by adding a terminating solution. By measuring the absorbance at 450 nm, the amount of p24 antigen was determined from a calibration curve prepared using standards attached to the kit.

2) Measurement of alkaline phosphatase activity

The alkaline phosphatase activity was measured by using Great EscAPe SEAP Detection Kit manufactured by Clontech. Fifty microliters of a diluent was added to 15 µl of the culture supernatant and incubated at 65°C for 30 minutes. The reaction solution was cooled to room temperature, and then 50 µl of the reaction solution was transferred to a white plate, and 50 µl of an assay buffer was added thereto and incubated at room temperature for 5 minutes. Fifty microliters of a luminescence substrate was added thereto, incubated at room temperature for 10 minutes, and measured for chemiluminescence by a luminometer.

Example 3. Evaluation of the anti-HIV-1 activity of a compound

A mixture of $2 \times 10^4$ of MOLT-4/CCR5/LTR-SEAP cells, 1000 $CCID_{50}$ (cell culture infective dose 50%) of the virus and a test compound (Table 1) at various concentrations was cultured at 37°C in 5% $CO_2$ gas for 5 days. A quarter of the culture was subcultured for 5 days. On the tenth day after the infection, the culture supernatant was recovered, and the alkaline phosphatase activity was measured. The anti-HIV-1 activity (the inhibition rate) of a test compound was calculated from the following formula:

Inhibition rate (%) =

$$(1 - (\text{chemiluminescence}^1/\text{chemiluminescence}^2)) \times 100$$

Chemiluminescence$^1$ = chemiluminescence with addition

of a test compound (ii) - chemiluminescence without viral

infection (iii).

Chemiluminescence$^2$ = chemiluminescence without

addition of a test compound (i) - chemiluminescence without

viral infection (iii).

Table 2 shows, as the anti-HIV-1 activity of the four compounds, an activity of inhibiting viral growth by 50% ($EC_{50}$) and an activity of inhibiting viral growth by 90% ($EC_{90}$) determined by using the alkaline phosphatase activity of the MOLT-4/CCR5/LTR-SEAP cells infected with a HIV-1 BaL strain as an index. As shown in Table 2, all the four compounds were recognized to have an anti-HIV-1 activity, the degree of which varied depending on the compounds.
The compounds used in this example are the four known compounds shown in Table 1.

[Table 1]

| 1. | Stavudine: 2',3'-didehydro-3'-deoxythymidine |
|---|---|
| 2. | Nevirapine: 11-cyclopropyl-5,11-dihydro-4-methyl-6H-dipyrido[3,2-b:2',3'-e][1,4]diazepin-6-one |
| 3. | Nelfinavir mesilate: (-)-(33,4aS,8aS)-N-tert-butyl-2-[(2R,3R)-2-hydroxy-3-(3-hydroxy-2-methylbenzoylamino)-4-(phenylthio)butyl]decahydroisoquinoline-3-carboxamide monomethanesulfonate |
| 4. | TAK-779: N,N-dimethyl-N-(4-(((2-(4-methylphenyl)-6,7dihydro-5H-benzocyclohepten-8-yl) carbonyl) amino) benzyl)N-(4-tetrahydropyranyl) ammonium chloride [which is a compound described in JP-A 2000-128842 and US6096780 and can be produced by methods described therein.] |

[Table 2]

| Compound | $EC_{50}$ (nN) | $EC_{90}$ (nM) |
|---|---|---|
| 1 | 16 | 260 |
| 2 | 40 | 350 |
| 3 | 0.67 | 170 |
| 4 | 21 | 680 |

Industrial Applicability

The T cell line according to the present invention carries a reporter gene that contains an LTR sequence of HIV and expresses CCR5, so that HIV can cause multi-round infection in said T cell line, and further said reporter gene facilitates the measurement of the degree of the multi-round infection in said T cell line. Accordingly, the T cell line of the present invention is useful for screening of compounds having prophylactic and therapeutic effects on diseases caused by HIV such as AIDS, or salts thereof.
Sequence Numbers in the Sequence Listing in this specification indicate the following sequences.
SEQ ID NO:1 shows an LTR sequence obtained in Example 1 (2).
SEQ ID NO:2 shows primer A used in Example 1 (2).
SEQ ID NO:3 shows primer B used in Example 1 (2).

**Claims**

1. A T cell line carrying a reporter gene that contains an LTR sequence of HIV and expressing CCR5.

2. The T cell line according to claim 1, wherein the LTR sequence is a nucleotide sequence identical or substantially identical with the nucleotide sequence set forth in SEQ ID NO:1.

3. The T cell line according to claim 1, wherein the reporter gene is an enzyme gene.

4. The T cell line according to claim 3, wherein the enzyme is alkaline phosphatase.

5. The T cell line according to claim 1, wherein the T cell line is a human-derived cell line.

6. The T cell line according to claim 1, wherein the T cell line is derived from a MOLT-4/CCR5 cell line (FERM BP-7060).

7. The T cell line according to claim 1, which is a MOLT-4/CCR5/LTR-SEAP cell line (FERM BP-7350).

8. A method of measuring the efficiency of infection with HIV, which comprises using the T cell line according to claim 1.

9. The method according to claim 8, wherein the HIV is HIV-1.

10. The method according to claim 8, wherein the HIV is R5 HIV-1.

11. The method according to claim 8, wherein the HIV is HIV present in a clinical sample.

12. A method of screening a compound that changes the efficiency of infection with HIV, which comprises culturing the T cell line according to claim 1 in the presence of a test compound and assaying a change in the efficiency of infection with HIV.

13. A method of assaying the reactivity for cell membrane fusion induced by mixing and culturing (1) the T cell line according to claim 1 and (2) a cell expressing (i) an HIV envelope glycoprotein and (ii) a transcription factor for activating HIV LTR.

14. A method of screening a compound that changes the reactivity for cell membrane fusion induced by mixing and culturing (1) the T cell line according to claim 1 and (2) a cell expressing (i) an HIV envelope glycoprotein and (ii) a transcription factor for activating HIV LTR, which comprises assaying the reactivity for cell membrane fusion induced by mixing and culturing the cells in the presence of a test compound.

15. A method of screening a compound that acts on HIV LTR, which comprises using the T cell line according to claim 1.

16. A method of screening a compound that inhibits activated HIV LTR, which comprises using the T cell line according to claim 1.

17. The method according to claim 16, wherein the HIV LTR is HIV LTR activated by HIV Tat.

18. The method according to claim 16, wherein the HIV LTR is HIV LTR activated by NFκB.

19. A compound that changes the efficiency of infection with HIV, which is obtained by the method according to claim 12.

20. The compound according to claim 19, which inhibits the efficiency of infection with HIV.

21. The compound according to claim 19, which promotes the efficiency of infection with HIV.

22. A compound that changes the reactivity for .cell membrane fusion, which is obtained by the method according to claim 14.

23. The compound according to claim 22, which inhibits cell membrane fusion.

**24.** The compound according to claim 22, which promotes cell membrane fusion.

**25.** A compound that acts on HIV LTR, which is obtained by the method according to claim 15.

**26.** The compound according to claim 25, which activates HIV LTR.

**27.** The compound according to claim 25, which inhibits HIV LTR.

**28.** A compound that inhibits activated HIV LTR, which is obtained by the method according to claim 16.

**29.** A compound that inhibits activated HIV LTR, which is obtained by the method according to claim 17.

**30.** A compound that inhibits activated HIV LTR, which is obtained by the method according to claim 18.

**31.** An agent for promoting the efficiency of infection with a retrovirus vector, which comprises the compound according to claim 21, 24 or 26.

**32.** The agent according to claim 31, wherein the retrovirus vector is derived from HIV.

**33.** A pharmaceutical composition comprising the compound according to any one of claims 19, 20, 22, 23, 25, 27, 28, 29 and 30.

**34.** The composition according to claim 33, which is an anti-HIV agent.

**35.** The composition according to claim 33, which is an agent for preventing or treating AIDS.

**36.** Use of the compound according to any one of claims 19, 20, 22, 23, 25, 27, 28, 29 and 30, or a salt thereof, for the production of an agent for preventing or treating AIDS.

**37.** A method of preventing or treating AIDS, which comprises administering a pharmacologically effective amount of the compound according to any one of claims 19, 20, 22, 23, 25, 27, 28, 29 and 30 or a salt thereof.

**38.** A method of evaluating the effect of combination use of two or more compounds having an anti-HIV activity.

Fig. 1

Fig. 2

Fig. 3

Fig. 4

BaL

Fig. 5

## INTERNATIONAL SEARCH REPORT

| | International application No. |
| --- | --- |
| | PCT/JP01/10018 |

A. CLASSIFICATION OF SUBJECT MATTER
Int.Cl⁷ C12N5/10, C12N15/11, C12Q1/02, C12Q1/70, A61P31/18

According to International Patent Classification (IPC) or to both national classification and IPC

B. FIELDS SEARCHED

Minimum documentation searched (classification system followed by classification symbols)
Int.Cl⁷ C12N5/10, C12N15/11, C12Q1/02, C12Q1/70, A61P31/18

Documentation searched other than minimum documentation to the extent that such documents are included in the fields searched

Electronic data base consulted during the international search (name of data base and, where practicable, search terms used)
WPIDS/BIOSIS/BIOTECHABS/MEDLINE/CA (STN), JICST (JOIS)

C. DOCUMENTS CONSIDERED TO BE RELEVANT

| Category* | Citation of document, with indication, where appropriate, of the relevant passages | Relevant to claim No. |
| --- | --- | --- |
| X Y | Baba, M. et al., "A small-molecule, nonpeptide CCR5 antagonist with highly potent and selective anti-HIV-1 activity", Proc. Natl. Acad. Sci. USA, (1999), Vol.96, pages 5698 to 5703 | 19,20,33-36 1-18,38 |
| X Y | Gervaix, A. et al., "A new reporter cell line to monitor HIV infection and drug susceptibility in vitro", Proc. Natl. Acad. Sci. USA, (1997), Vol.94, pages 4653 to 4658 | 19,20,33-36 1-18,38 |
| Y A | Takashi KURIMURA, "Nyuumon 'Aids-gaku'", Kabushiki Kaisha Kagaku Doujin, (1997), pages 139 to 149 | 38 1-20,33-36 |

☐ Further documents are listed in the continuation of Box C. ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search 12 February, 2002 (12.02.02) | Date of mailing of the international search report 26 February, 2002 (26.02.02) |
| --- | --- |
| Name and mailing address of the ISA/ Japanese Patent Office | Authorized officer |
| Facsimile No. | Telephone No. |

Form PCT/ISA/210 (second sheet) (July 1992)

**EP 1 335 019 A1**

## INTERNATIONAL SEARCH REPORT

International application No.

PCT/JP01/10018

---

**Box I    Observations where certain claims were found unsearchable (Continuation of item 1 of first sheet)**

This international search report has not been established in respect of certain claims under Article 17(2)(a) for the following reasons:

1. ☒   Claims Nos.: 37
   because they relate to subject matter not required to be searched by this Authority, namely:

   It pertains to methods for treatment of the human body by surgery
   or therapy as well as diagnostic methods.

2. ☒   Claims Nos.: 19-36
   because they relate to parts of the international application that do not comply with the prescribed requirements to such an
   extent that no meaningful international search can be carried out, specifically:

   (See extra sheet.)

3. ☐   Claims Nos.:
   because they are dependent claims and are not drafted in accordance with the second and third sentences of Rule 6.4(a).

---

**Box II    Observations where unity of invention is lacking (Continuation of item 2 of first sheet)**

This International Searching Authority found multiple inventions in this international application, as follows:

The invention as set forth in claim 38 does not involve "a T cell
line carrying a reporter gene which has the LTR sequence of HIV and expressing
CCR5", i.e., the technical matter common to the inventions as set forth
in claims 1 to 36. Moreover, it is not recognized that there is a novel
problem common to them.
   Such being the case, there is no technical relevancy in the meaning as
defined in PCT Rule 13.2 between the group of the inventions as set forth
in claims 1 to 36 and the invention as set forth in claim 38 and thus the
requirement of unity of invention is not fulfilled thereby.

1. ☒   As all required additional search fees were timely paid by the applicant, this international search report covers all searchable
   claims.

2. ☐   As all searchable claims could be searched without effort justifying an additional fee, this Authority did not invite payment
   of any additional fee.

3. ☐   As only some of the required additional search fees were timely paid by the applicant, this international search report covers
   only those claims for which fees were paid, specifically claims Nos.:

4. ☐   No required additional search fees were timely paid by the applicant. Consequently, this international
   search report is restricted to the invention first mentioned in the claims; it is covered by claims Nos.:

**Remark on Protest**    ☐    The additional search fees were accompanied by the applicant's protest.

☒    No protest accompanied the payment of additional search fees.

---

Form PCT/ISA/210 (continuation of first sheet (1)) (July 1992)

| INTERNATIONAL SEARCH REPORT | International application No. |
|---|---|
|  | PCT/JP01/10018 |

Continuation of Box No.I-2 of continuation of first sheet(1)

　　　　Concerning the inventions as set forth in claims 19, 20 and 33 to 36, it is not stated what compounds are involved in the scope of the compound inhibiting the HIV infection efficiency other than the four compounds listed in Table 1 in the description. Thus, it is completely unknown what compounds other than the four compounds listed in Table 1 in the description are involved in the scope thereof. Such being the case, the inventions as set forth in the above claims are not clear to such an extent as ensuring a meaningful international search. Therefore, this international search has been practiced exclusively on the four compounds listed in Table 1.

　　　　Concerning the inventions as set forth in claims 21 to 36, it is completely unknown what compounds are involved in the scopes of the compound elevating the HIV infection efficiency, the compound changing the reactivity in cell membrane fusion, the compound acting on HIV LTR and the compound suppressing activated HIV LTR. Such being the case, the inventions as set forth in these claims are not clear to such an extent as ensuring a meaningful international search.

Form PCT/ISA/210 (extra sheet) (July 1992)

SEQUENCE LISTING

<110> Takeda Chemical Industries, Ltd.

<120> T-lymphoblastoid cell line and Use Thereof

<130> 181124

<150> JP 2000-350685

<151> 2000-11-17

<160> 3

<210> 1

<211> 220

<212> DNA

<213> Virus

<400> 1

CTTCAAGAAC TGCTGACATC GAGCTTGCTA CAAGGGACTT TCCGCTGGGG ACTTTCCAGG        60

GAGGCGTGGC CTGGGCGGGA CTGGGGAGTG GCGAGCCCTC AGATGCTGCA TATAAGCAGC       120

TGCTTTTTGC CTGTACTGGG TCTCTCTGGT TAGACCAGAT CTGAGCCTGG GAGCTCTCTG       180

GCTAACTAGG GAACCCACTG CTTAAGCCTC AATAAAGCTT                             220

<210> 2

<211> 30

<212> DNA

<213> Artificial Sequence

<400> 2

GGAGATCTTC AAGAACTGCT GACATCGAGC                                         30

<210> 3

<211> 30

<212> DNA

<213> Artificial Sequence

<400> 3

AGGCAAGCTT TATTGAGGCT TAAGCAGTGG                                         30

14